# EUROPÄISCHE PATENTSCHRIFT

(11) **EP 1 902 033 B1**
(45) Veröffentlichungstag und Bekanntmachung des Hinweises auf die Patenterteilung: **12.08.2009**
(21) Anmeldenummer: 06754192.0
(22) Anmeldetag: 07.06.2006
(51) Int. Cl.: C07D 231/16

(54) **VERFAHREN ZUM HERSTELLEN VON CARBOXAMIDEN**
METHOD FOR PRODUCING CARBOXAMIDES
PROCEDE DE PRODUCTION DE CARBOXAMIDES

(30) Priorität: 18.06.2005 DE 102005028293
(43) Veröffentlichungstag der Anmeldung: 26.03.2008
(73) Patentinhaber: Bayer CropScience AG, 40789 Monheim (DE)
(72) Erfinder: NEEFF, Arnd, 58256 Ennepetal (DE); PAZENOK, Sergiy, 42699 Solingen (DE)
(86) Internationale Anmeldenummer: PCT/EP2006/005435
(87) Internationale Veröffentlichungsnummer: WO 2006/136287

(56) Entgegenhaltungen:
- EP-A- 0 776 889
- WO-A-20/06092291

## Beschreibung

Die vorliegende Erfindung betrifft ein neues Verfahren zum Herstellen von bekannten fungizid wirksamen 1,3-Dimethyl-5-fluor-1H-pyrazol-4-carboxamiden aus dem entsprechenden Säurefluorid und Anilin-Derivaten, in Abwesenheit eines Säureakzeptors.

Es ist bereits bekannt, dass man 1,3-Dimethyl-5-fluor-1H-pyrazol-4-carboxamide durch Umsetzung des entsprechenden Säurefluorids mit dem gewünschten Anilin-Derivat erhält (vgl. EP-A 0 776 889). Bevorzugt werden nach dieser Beschreibung bicyclische tertiäre Amine wie Diazabicyclooctan (DABCO), Diazabicyclononen (DBN) oder Diazabicycloundecen (DBU) als Säureakzeptor eingesetzt. Die Umsetzung mit DABCO liefert lediglich eine Ausbeute von 80 %. Außerdem ist DABCO für großtechnische Umsetzungen ungeeignet, das dieses Reagenz sehr teuer und nicht zu recyclieren ist.

Es wurde nun gefunden, dass man Carboxamide der Formel (I) 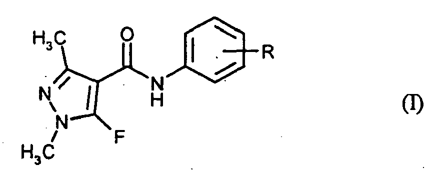 in welcher
- R: für C₃-C₁₂-Cycloalkyl, C₃-C₁₂-Cycloalkenyl, C₆-C₁₂-Bicycloalkyl, C₂-C₁₂-Oxacycloalkyl, C₄-C₁₂-Oxacycloalkenyl, C₃-C₁₂-Thiacycloalkyl, C₄-C₁₂-Thiacycloalkenyl, C₂-C₁₂-Azacycloalkyl steht, welche jeweils gegebenenfalls einfach oder mehrfach, gleich oder verschieden durch C₁-C₈-Alkyl, C₁-C₈-Alkoxy, Halogen und/oder Cyano substituiert sein können,
oder für gegebenenfalls einfach bis fünffach, gleich oder verschieden substituiertes Phenyl steht, wobei die Substituenten jeweils aus der Liste W¹ ausgewählt sind,
oder für unsubstituiertes C₂-C₂₀-Alkyl,
oder für einfach oder mehrfach, gleich oder verschieden durch Halogen, C₁-C₆-Alkylthio, C₁-C₆-Alkylsulfinyl, C₁-C₆-Alkylsulfonyl, C₁-C₆-Alkoxy, C₁-C₆-Alkylamino, Di(C₁-C₆-alkyl)amino, C₁-C₆-Halogenalkylthio, C₁-C₆-Halogenalkylsulfinyl, C₁-C₆-Halogenalkylsulfonyl, C₁-C₆-Halogenalkoxy, C₁-C₆-Halogenalkylamino, Halogen-di(C₁-C₆-alkyl)amino, -SiR¹R²R³ und/oder C₃-C₆-Cycloalkyl substituiertes C₁-C₂₀-Alkyl steht, wobei der Cycloalkylteil seinerseits gegebenenfalls einfach bis vierfach, gleich oder verschieden durch Halogen, C₁-C₄-Alkyl und/oder C₁-C₄-Halogenalkyl substituiert sein kann,
- W¹: für Halogen, Cyano, Nitro, Amino, Hydroxy, C₁-C₈-Alkyl, C₁-C₈-Alkoxy, C₁-C₈-Alkylthio, C₁-C₈-Alkylsulfinyl, C₁-C₈-Alkylsulfonyl, C₂-C₆-Alkenyl, C₂-C₆-Alkenyloxy; C₁-C₆-Halogenalkyl, C₁-C₆-Halogenalkoxy, C₁-C₆-Halogenalkylthio, C₁-C₆-Halogenalkylsulfiriyl oder C₁-C₆-Halogenalkylsulfonyl mit jeweils 1 bis 13 gleichen oder verschiedenen Halogenatomen; C₂-C₆-Halogenalkenyl oder C₂-C₆-Halogenalkenyloxy mit jeweils 1 bis 11 gleichen oder verschiedenen Halogenatomen; C₃-C₆-Cycloalkyl oder C₃-C₆-Cycloalkyloxy;
- R¹ und R²: unabhängig voneinander für Wasserstoff, C₁-C₈-Alkyl, C₁-C₈-Alkoxy, C₁-C₄-Alkoxy-C₁-C₄-alkyl, C₁-C₄-Alkylthio-C₁-C₄-alkyl oder C₁-C₆-Halogenalkyl stehen,
- R³: für Wasserstoff, C₁-C₈-Alkyl, C₁-C₈-Alkoxy, C₁-C₄-Alkoxy-C₁-C₄-alkyl, C₁-C₄-Alkylthio-C₁-C₄-alkyl, C₂-C₈-Alkenyl, C₂-C₈-Alkinyl, C₁-C₆-Halogenalkyl, C₂-C₆₋Halogenalkenyl, C₂-C₆-Halogenalkinyl, C₃-C₆-Cycloalkyl, oder für jeweils gegebenenfalls substituiertes Phenyl oder Phenylalkyl steht,
auf einfache Weise erhält, indem man

5-Fluor-1,3-dimethyl-1H-pyrazol-4-carbonsäurefluorid der Formel (II) 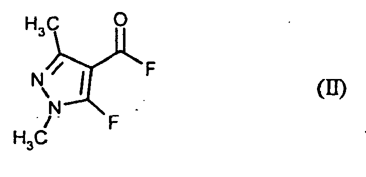 mit Anilin-Derivaten der Formel (III) 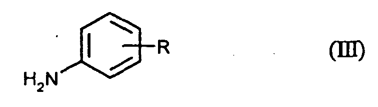 in welcher R die oben angegebenen Bedeutungen hat,
in Abwesenheit eines Säureakzeptors umsetzt.

Überraschenderweise lassen sich die Carboxamide der Formel (I) unter den erfindungsgemäßen Bedingungen mit guten Ausbeuten in hoher Reinheit und Selektivität herstellen. Ein weiterer Vorteil des erfindungsgemäßen Verfahrens ist, dass sich die Aufarbeitung einfacher gestaltet, da auf den Einsatz eines Säureakzeptors verzichtet wird. Das erfindungsgemäße Verfahren wird dadurch außerdem wirtschaftlicher. Zudem kann die Reaktionszeit verkürzt werden.

Verwendet man beispielsweise 5-Fluor-1,3-dimethyl-1H-pyrazol-4-carbonsäurefluorid und 2-(1,3-Dimethyl-butyl)-phenylamin als Ausgangsstoffe, so kann das erfindungsgemäße Verfahren durch das folgende Formelschema veranschaulicht werden:

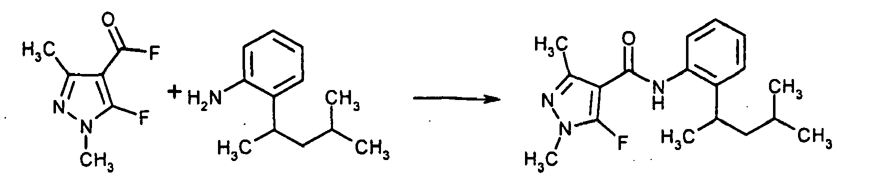

Das bei der Durchführung des erfindungsgemäßen Verfahrens als Ausgangsstoff verwendete 5-Fluor-1,3-dimethyl-1H-pyrazol-4-carbonsäurefluorid der Formel (II) ist bekannt (vgl. EP-A 0 776 889).

Die bei der Durchführung des erfindungsgemäßen Verfahrens weiterhin als Ausgangsstoffe verwendeten Anilin-Derivate sind durch die Formel (III) allgemein definiert.
- R: steht bevorzugt für C₃-C₈-Cycloalkyl, C₃-C₈-Cycloalkenyl, C₆-C₁₀-Bicycloalkyl, C₂-C₇-Oxacycloalkyl, C₄-C₇-Oxacycloalkenyl, C₃-C₇-Thiacycloalkyl, C₄-C₇-Thiacycloalkenyl, C₂-C₇-Azacycloalkyl steht, welche jeweils gegebenenfalls einfach bis vierfach, gleich oder verschieden durch C₁-C₄-Alkyl, C₁-C₄-Alkoxy, Fluor, Chlor, Brom und/oder Cyano substituiert sein können,
oder für einfach bis dreifach, gleich oder verschieden substituiertes Phenyl, wobei die Substituenten aus der Liste W¹ ausgewählt sind,
oder für unsubstituiertes C₂-C₁₂-Alkyl (wie Ethyl und geradkettiges oder verzweigtes Propyl, Butyl, Pentyl, Hexyl, Heptyl, Octyl, Nonyl, Decyl, Undecyl und Dodecyl)
oder für einfach oder mehrfach, gleich oder verschieden durch Fluor, Chlor, Brom, Iod, C₁-C₆-Alkylthio, C₁-C₄-Alkylsulfinyl, C₁-C₄-Alkylsulfonyl, C₁-C₄-Alkoxy, C₁-C₄-Alkylamino, Di(C₁-C₄-alkyl)amino, C₁-C₄-Halogenalkylthio, C₁-C₄-Halogenalkylsulfinyl, C₁-C₄-Halogenalkylsulfonyl, C₁-C₄-Halogenalkoxy, C₁-C₄-Halogenalkylamino, Halogen-di(C₁-C₄-alkyl)amino mit jeweils 1 bis 9 Fluor-, Chlor- und/oder Bromatomen, -SiR¹R²R³, Cyclopropyl, Dichlorcyclopropyl, Cyclobutyl, Cyclopentyl oder Cyclohexyl substituiertes C₁-C₁₂-Alkyl (wie Methyl, Ethyl und geradkettiges oder verzweigtes Propyl, Butyl, Pentyl, Hexyl, Heptyl, Octyl, Nonyl, Decyl, Undecyl und Dodecyl).
- R: steht besonders bevorzugt für Cyclopentyl, Cyclohexyl, Cycloheplyl, Bicyclo[2.2.1 heptyl, oder für einfach in 4-Position substituiertes Phenyl, für zweifach, gleich oder verschieden in 3,4-, 2,3-, 2,4- oder 3,5-Position substituiertes Phenyl oder für dreifach, gleich oder verschieden in 2,4,6-Position substituiertes Phenyl, wobei die Substituenten jeweils aus der Liste W¹ ausgewählt sind, oder für unsubstituiertes C₃-C₁₀-Alkyl (wie Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-3-methylpropyl, n-Heptyl, 1-Methylhexyl, 1-Ethylpentyl, 2-Ethylpentyl, 1-Propylbutyl, Octyl, 1-Methylheptyl, 2-Methylheptyl, 1-Ethylhexyl, 2-Ethylhexyl, 1-Propylpentyl, 2-Propylpentyl, Nonyl, 1-Methyloctyl, 2-Methyloctyl, 1-Ethylheptyl, 2-Ethylheptyl, 1-Propylhexyl, 2-Propylhexyl, Decyl, 1-Methylnonyl, 2-Methylnonyl, 1-Ethyloctyl, 2-Ethyloctyl, 1-Propylheptyl und 2-Propylheptyl) oder für einfach oder mehrfach, gleich oder verschieden durch Fluor, Chlor, Methylthio, Ethylthio, n- oder iso-Propylthio, n-, iso-, sec-, tert-Butylthio, Pentylthio, Hexylthio, Methylsulfonyl, Ethylsulfonyl, n- oder iso-Propylsulfonyl, n-, iso-, sec-, tert-Butylsulfonyl, Methoxy, Ethoxy, n- oder iso-Propoxy, n-, iso-, sec-, tert-Butoxy, Methylamino, Ethylamino, n- oder iso-Propylamino, n-, iso-, sec-, tert-Butylamino, Dimethylamino, Diisopropylamino, Trifluormethylthio, Trifluormethoxy, -SiR¹R²R³, Cyclopropyl, Dichlorcyclopropyl, Cyclobutyl, Cyclopentyl oder Cyclohexyl substituiertes C₁-C₁₀-Alkyl (wie Methyl, Ethyl,- Propyl, 1-Methylethyl, Butyl, 1-Methylpropyl, 2-Methylpropyl, 1,1-Dimethylethyl, n-Pentyl,1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbütyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-3-methylpropyl, n-Heptyl, 1-Methylhexyl, 1-Ethylpentyl, 2-Ethylpentyl, 1-Propylbutyl, Octyl, 1-Methylheptyl, 2-Methylheptyl, 1-Ethylhexyl, 2-Ethylhexyl, 1-Propylpentyl, 2-Propylpentyl, Nonyl, 1-Methyloctyl, 2-Methyloctyl, 1-Ethylheptyl, 2-Ethylheptyl, 1-Propylhexyl, 2-Propylhexyl, Decyl, 7-Methylnonyl, 2-Methylnonyl, 1-Ethyloctyl, 2-Ethyloctyl, 1-Propylheptyl und 2-Propylheptyl).
- W¹: steht bevorzugt für Fluor, Chlor, Brom, Methyl, Ethyl, n- oder i-Propyl, n-, i-, s- oder t-Butyl, Methoxy, Ethoxy, n- oder i-Propoxy, Trifluormethyl, Trifluorethyl, Difluormethoxy, Trifluormethoxy, Difluorchlormethoxy, Trifluorethoxy.
- W¹: steht besonders bevorzugt Fluor, Chlor oder Brom.
- R¹ und R²: stehen unabhängig voneinander bevorzugt für C₁-C₆-Alkyl, C₁-C₆-Alkoxy, C₁-C₃-Alkoxy-C₁-C₃-alkyl oder C₁-C₃-Alkylthio-C₁-C₃-alkyl.
- R¹ und R²: stehen unabhängig voneinander besonders bevorzugt für Methyl, Ethyl, Methoxy, Ethoxy, Methoxymethyl, Ethoxymethyl, Methoxyethyl, Ethoxyethyl, Methylthiomethyl, Ethylthiomethyl, Methylthioethyl oder Ethylthioethyl.
- R¹ und R²: stehen unabhängig voneinander ganz besonders bevorzugt für Methyl, Methoxy, Methoxymethyl oder Methylthiomethyl.
- R¹ und R²: stehen insbesondere bevorzugt jeweils für Methyl.
- R³: steht bevorzugt für C₁-C₆-Alkyl, C₁-C₆-Alkoxy, C₁-C₃-Alkoxy-C₁-C₃-alkyl, C₁-C₃-Alkylthio-C₁-C₃-alkyl, C₃-C₆-Cycloalkyl, Phenyl oder Benzyl.
- R³: steht besonders bevorzugt für Methyl, Ethyl, n- oder iso-Propyl, n-, sec-, iso- oder tert-Butyl, Methoxy, Ethoxy, n- oder iso-Propoxy, n-, sec-, iso- oder tert-Butoxy, Methoxymethyl, Ethoxymethyl, Methoxyethyl, Ethoxyethyl, Methylthiomethyl, Ethylthiomethyl, Methylthioethyl, Ethylthioethyl, Cyclopropyl, Phenyl oder Benzyl.
- R³: steht ganz besonders bevorzugt für Methyl, Ethyl, n- oder iso-Propyl, iso- oder tert-Butyl, Methoxy, iso-Propoxy, iso- oder tert-Butoxy.
- R³: steht insbesondere bevorzugt für Methyl.

Bevorzugt werden Anilin-Derivate der Formel (III-1) 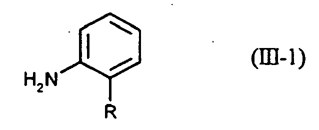 in welcher R die oben angegebenen Bedeutungen hat,
in das erfindungsgemäße Verfahren eingesetzt.

Außerdem bevorzugt werden Anilin-Derivate der Formel (III-2) 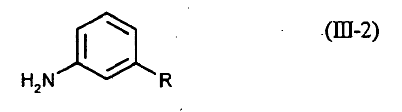 in welcher R die oben angegebenen Bedeutungen hat,
in das erfindungsgemäße Verfahren eingesetzt.

Außerdem bevorzugt werden Anilin-Derivate der Formel (III-3) 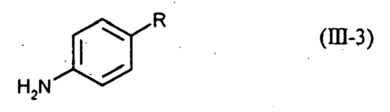 in welcher R die oben angegebenen Bedeutungen hat,
in das erfindungsgemäße Verfahren eingesetzt.

Besonders bevorzugt setzt man Anilin-Derivate der Formel (III-1) ein.

Anilin-Derivate der Formel (III) bzw. (III-1), (III-2) und (III-3) sind bekannt oder lassen sich auf bekannte Weise herstellen (vgl. EP-A 0 776 889, WO 03/010149).

Das erfindungsgemäße Verfahrens kann in Gegenwart eines Verdünnungsmittels durchgeführt werden. Hierzu kommen alle inerten organischen Lösungsmittel in Betracht, vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielswiese Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid, besonders bevorzugt Chlorbenzol oder Toluol.

Die Reaktionstemperaturen können bei der Durchführung des erfindungsgemäßen Verfahrens in einem größeren Bereich variiert werden. Im Allgemeinen arbeitet man bei Temperaturen von 120°C bis 150°C, bevorzugt bei Temperaturen von 130°C bis 140°C.

Bei der Durchführung des erfindungsgemäßen Verfahrens setzt man pro Mol des 5-Fluor-1,3-dimethyl-1H-pyrazol-4-carbonsäurefluorids der Formel (II) im Allgemeinen zwischen 0,8 und 1,5 Mol, bevorzugt äquimolare Mengen, von Anilin-Derivaten der Formel (III) ein.

Die Reaktionszeit kann in Abhängigkeit von der Reaktivität der Edukte bis zu 10 Stunden betragen, wobei der Abbruch der Reaktion bei vollständigem Umsatz auch schon früher erfolgen kann. Bevorzugt sind Reaktionszeiten von 5 Stunden.

Alle erfindungsgemäßen Verfahren werden im Allgemeinen unter Normaldruck durchgerührt. Es ist jedoch auch möglich; unter erhöhtem oder vermindertem Druck - im Allgemeinen zwischen 0,1 bar und 10 bar - zu arbeiten.

Die durch das erfindungsgemäßen Verfahren herstellbaren Carboxamide der Formel (I) sind wertvolle Fungizide (vgl, z.B. WO 03/010149).

Das erfindungsgemäße Herstellen von Carboxamiden der Formel (I) wird in den nachstehenden Beispielen beschrieben, welche die obige Beschreibung weiter illustrieren. Die Beispiele sind jedoch nicht in einschränkender Weise zu interpretieren.

### Herstellungsbeispiele

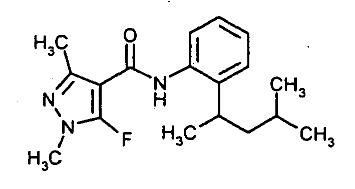

Unter Schutzgas (Argon) wird eine Lösung aus 2-(1,3-Dimethyl-butyl)-phenylamin 18,05 g (100 mmol) in 40 ml Chlorbenzol vorgelegt. Man gibt 16,17 g (100 mmol) 5-Fluor-1,3-dimethyl-1H-pyrazol-4-carbonsäurefluorid zu und rührt für 5 h bei 130°C nach. Zur Aufarbeitung lässt man abkühlen, gibt 100 ml Wasser zu und extrahiert dreimal mit je 100 ml Essigsäureethylester. Die vereinigten organischen Phasen werden einmal mit 100 ml Wasser gewaschen, über Magnesiumsulfat getrocknet und unter vermindertem Druck eingeengt. Die erhaltene Suspension wird mit 100 ml für 2 h bei Raumtemperatur nachgerührt Man erhält 28,2 g (89 % der Theorie) an N-[2-(1,3-Dimethylbutyl)phenyl]-5-fluor-1,3-dimethyl-1H-pyrazol-4-carboxamid in Form von Kristallen(Schmelzpunkt 104-106°C).

## Patentansprüche

1. Verfahren zum Herstellen von Carboxamiden der Formel (I) 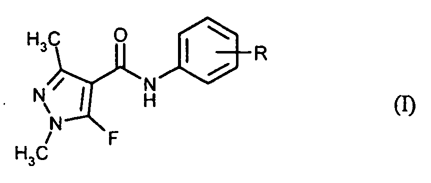 in welcher
R für C₁-C₁₂-Cycloalkyl, C₃-C₁₂-Cycloalkenyl, C₆-C₁₂-Bicycloalkyl, C₂-C₁₂-Oxacycloalkyl, C₄-C₁₂-Oxacycloalkenyl, C₃-C₁₂-Thiacycloalkyl, C₄-C₁₂-Thiacycloalkenyl, C₂-C₁₂-Azacycloalkyl steht, welche jeweils gegebenenfalls einfach oder mehrfach, gleich oder verschieden durch C₁-C₈-Alkyl, C₁-C₈-Alkoxy, Halogen und/oder Cyano substituiert sein können,
oder für gegebenenfalls einfach bis fünffach, gleich oder verschieden substituiertes Phenyl steht, wobei die Substituenten jeweils aus der Liste W¹ ausgewählt sind,
oder für unsubstituiertes C₂-C₂₀-Alkyl,
oder für einfach oder mehrfach, gleich oder verschieden durch Halogen, C₁-C₆-Alkylthio, C₁-C₆-Alkylsulfinyl, C₁-C₆-Alkylsulfonyl, C₁-C₆-Alkoxy, C₁-C₆-Alkylamino, Di(C₁-C₆-alkyl)amino, C₁-C₆-Halogenalkylthio, C₁-C₆-Halogenalkylsulfinyl, C₁-C₆-Halogenalkylsulfonyl, C₁-C₆-Halogenalkoxy, C₁-C₆-Halogenalkylamino, Halogen-di(C₁-C₆-alkyl)amino, -SiR¹R²R³ und/oder C₃-C₆-Cycloalkyl substituiertes C₁-C₂₀-Alkyl steht, wobei der Cycloalkylteil seinerseits gegebenenfalls einfach bis vierfach, gleich oder verschieden durch Halogen, C₁-C₄-Alkyl und/oder C₁-C₄-Halogenalkyl substituiert sein kann,
W¹ für Halogen, Cyano, Nitro, Amino, Hydroxy, C₁-C₈-Alkyl, C₁-C₈-Alkoxy, C₁-C₈₋Alkylthio, C₁-C₈-Alkylsulfinyl, C₁-C₈-Alkylsulfonyl, C₂-C₆-Alkenyl, C₂-C₆-Alkenyloxy; C₁-C₆-Halogenalkyl, C₁-C₆-Halogenalkoxy, C₁-C₆-Halogenalkylthio, C₁-C₆-Halogenalkylsulfinyl oder C₁-C₆-Halogenalkylsulfonyl mit jeweils 1 bis 13 gleichen oder verschiedenen Halogenatomen; C₂-C₆-Halogenalkenyl oder C₂-C₆-Halogenalkenyloxy mit jeweils 1 bis 11 gleichen oder verschiedenen Halogenatomen; C₃-C₆-Cycloalkyl oder C₃-C₆-Cycloalkyloxy;
R¹ und R² unabhängig voneinander für Wasserstoff, C₁-C₈-Alkyl, C₁-C₈-Alkoxy, C₁-C₄-Alkoxy-C₁-C₄-alkyl, C₁-C₄-Alkylthio-C₁-C₄-alkyl oder C₁-C₆-Halogenalkyl stehen,
R³ für Wasserstoff, C₁-C₈-Alkyl, C₁-C₈-Alkoxy, C₁-C₄-Alkoxy-C₁-C₄-alkyl, C₁-C₄-Alkylthio-C₁-C₄-alkyl, C₂-C₈-Alkenyl, C₂-C₈-Alkinyl, C₁-C₆-Halogenalkyl, C₂-C₆-Halogenalkenyl, C₂-C₆-Halogenalkinyl, C₃-C₆-Cycloalkyl, oder für jeweils gegebenenfalls substituiertes Phenyl oder Phenylalkyl steht,
**dadurch gekennzeichnet, dass** man
5-Fluor-1,3-dimethyl-1H-pyrazol-4-carbonsäurefluorid der Formel (II) 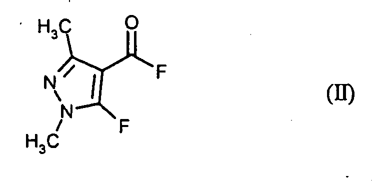 mit Anilin-Derivaten der Formel (III) 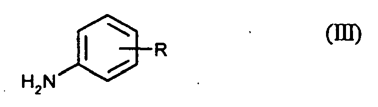 in welcher R die oben angegebenen Bedeutungen hat,
in Abwesenheit eines Säureakzeptors umsetzt.

2. Verfahren gemäß Anspruch 1, **dadurch gekennzeichnet, dass** man bei Temperaturen von 120°C bis 150°C arbeitet.

3. Verfahren gemäß Anspruch 1 oder 2, **dadurch gekennzeichnet, dass** man Anilin-Derivate der Formel (III-1) 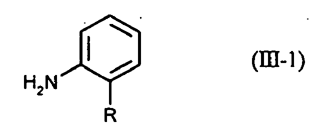 in welcher R die in Anspruch 1 angegebenen Bedeutungen hat, einsetzt.

4. Verfahren gemäß Anspruch 1, 2 oder 3, **dadurch gekennzeichnet, dass** man 2-(1,3-Dimethyl-butyl)-phenylamin als Anilin-Derivat der Formel (III) einsetzt.

## Claims

1. Process for preparing carboxamides of the formula (I) 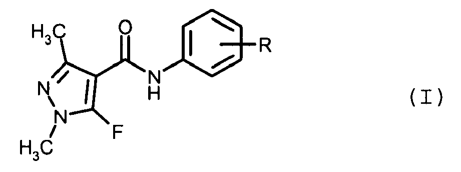 in which
R is C₃-C₁₂-cycloalkyl, C₃-C₁₂-cycloalkenyl, C₆-C₁₂-bicycloalkyl, C₂-C₁₂-oxacycloalkyl, C₄-C₁₂-oxacycloalkenyl, C₃-C₁₂-thiacycloalkyl, C₄-C₁₂-thiacycloalkenyl, C₂-C₁₂-azacycloalkyl, each of which may optionally be mono- or polysubstituted, identically or differently, by C₁-C₈-alkyl, C₁-C₈-alkoxy, halogen and/or cyano, or phenyl which is optionally mono- to pentasubstituted identically or differently, where the substituents are each selected from the list W¹,
or unsubstituted C₂-C₂₀-alkyl,
or C₁-C₂₀-alkyl which is mono- or polysubstituted, identically or differently, by halogen, C₁-C₆-alkylthio, C₁-C₆-alkylsulfinyl, C₁-C₆-alkylsulfonyl, C₁-C₆-alkoxy, C₁-C₆-alkylamino, di(C₁-C₆-alkyl)amino, C₁-C₆-haloalkylthio, C₁-C₆-haloalkylsulfinyl, C₁-C₆-haloalkylsulfonyl, C₁-C₆-haloalkoxy, C₁-C₆-haloalkylamino, halodi(C₁-C₆-alkyl)amino, -SiR¹R²R³ and/or C₃-C₆-cycloalkyl, where the cycloalkyl moiety may in turn optionally be mono- to tetrasubstituted, identically or differently, by halogen, C₁-C₄-alkyl and/or C₁-C₄-haloalkyl,
W¹ is halogen, cyano, nitro, amino, hydroxyl, C₁-C₈-alkyl, C₁-C₈-alkoxy, C₁-C₈-alkylthio, C₁-C₈-alkylsulfinyl, C₁-C₈-alkylsulfonyl, C₂-C₆-alkenyl, C₂-C₆-alkenyloxy; C₁-C₆-haloalkyl, C₁-C₆-haloalkoxy, C₁-C₆-haloalkylthio, C₁-C₆-haloalkylsulfinyl or C₁-C₆-haloalkylsulfonyl having in each case from 1 to 13 identical or different halogen atoms; C₂-C₆-haloalkenyl or C₂-C₆-haloalkenyloxy having in each case from 1 to 11 identical or different halogen atoms; C₃-C₆-cycloalkyl or C₃-C₆-cycloalkyloxy;
R¹ and R² are each independently hydrogen, C₁-C₈-alkyl, C₁-C₈-alkoxy, C₁-C₄-alkoxy-C₁-C₄-alkyl, C₁-C₄-alkylthio-C₁-C₄-alkyl or C₁-C₆-haloalkyl,
R³ is hydrogen, C₁-C₈-alkyl, C₁-C₈-alkoxy, C₁-C₄-alkoxy-C₁-C₄-alkyl, C₁-C₄-alkylthio-C₁-C₄-alkyl, C₂-C₈-alkenyl, C₂-C₈-alkynyl, C₁-C₆-haloalkyl, C₂-C₆-haloalkenyl, C₂-C₆-haloalkynyl, C₃-C₆-cycloalkyl, or in each case optionally substituted phenyl or phenylalkyl,
**characterized in that**
5-fluoro-1,3-dimethyl-1H-pyrazole-4-carbonyl fluoride of the formula (II) 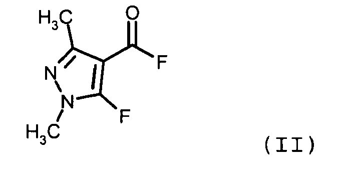 is reacted with aniline derivatives of the formula (III) 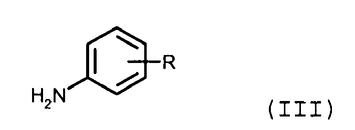
in which R is as defined above,
in the absence of an acid acceptor.

2. Process according to Claim 1, **characterized in that** temperatures of from 120°C to 150°C are employed.

3. Process according to Claim 1 or 2, **characterized in that** aniline derivatives of the formula (III-1) 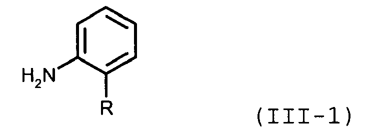 in which R is as defined in Claim 1 are used.

4. Process according to Claim 1, 2 or 3, **characterized in that** 2-(1,3-dimethylbutyl)phenylamine is used as the aniline derivative of the formula (III).

## Revendications

1. Procédé de production de carboxamides de formule (I) 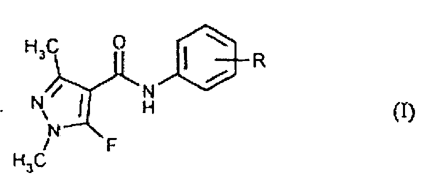 dans laquelle
R représente un reste cycloalkyle en C₃ à C₁₂, cycloalcényle en C₃ à C₁₂, bicycloalkyle en C₆ à C₁₂, oxacycloalkyle en C₂ à C₁₂, oxacycloalcényle en C₄ à C₁₂, thiacycloalkyle en C₃ à C₁₂, thiacycloalcényle en C₄ à C₁₂, azacycloalkyle en C₂ à C₁₂, qui peuvent être éventuellement substitués dans chaque cas une ou plusieurs fois identiques ou différentes par un radical alkyle en C₁ à C₈, alkoxy en C₁ à C₈, halogéno et/ou cyano,
ou un reste phényle éventuellement substitué une à cinq fois identiques ou différentes, les substituants étant choisis à chaque fois dans la liste W¹,
ou un reste alkyle en C₂ à C₂₀ non substitué,
ou un reste alkyle en C₁ à C₂₀ substitué une ou plusieurs fois identiques ou différentes par un radical halogéno, alkylthio en C₁ à C₆, alkylsulfinyle en C₁ à C₆, alkylsulfonyle en C₁ à C₆, alkoxy en C₁ à C₆, alkylamino en C₁ à C₆, di(alkyle en C₁ à C₆)amino, halogénalkylthio en C₁ à C₆, halogénalkylsulfinyle en C₁ à C₆, halogénalkylsulfonyle en C₁ à C₆, halogénalkoxy en C₁ à C₆, halogénalkylamino en C₁ à C₆, halogéno-di(alkyle en C₁ à C₆) amino, -SiR¹R²R³ et/ou cycloalkyle en C₃ à C₆, la partie cycloalkyle pouvant elle-même éventuellement être substituée une ou plusieurs fois identiques ou différentes par un radical halogéno, alkyle en C₁ à C₄ et/ou halogénalkyle en C₁ à C₄,
W¹ représente un reste halogéno, cyano, nitro, amino, hydroxy, alkyle en C₁ à C₈, alkoxy en C₁ à C₈, alkylthio en C₁ à C₈, alkylsulfinyle en C₁ à C₈, alkylsulfonyle en C₁ à C₈, alcényle en C₂ à C₆, alcényloxy en C₂ à C₆ ; halogénalkyle en C₁ à C₆, halogénalkoxy en C₁ à C₆, halogénalkylthio en C₁ à C₆, halogénalkylsulfinyle en C₁ à C₆ ou halogénalkylsulfonyle en C₁ à C₆ ayant chacun 1 à 13 atomes d'halogènes identiques ou différents ; halogénalcényle en C₂ à C₆ ou halogénalcényloxy en C₂ à C₆ ayant chacun 1 à 13 atomes d'halogènes identiques
ou différents; halogénalcényle en C₂ à C₆ ou halogénalcényloxy en C₂ à C₆ ayant chacun 1 à 11 atomes d'halogènes identiques ou différents ; cycloalkyle en C₃ à C₆ ou cycloalkyloxy en C₃ à C₆ ;
R¹ et R² représentent indépendamment l'un de l'autre l'hydrogène, un reste alkyle en C₁ à C₈, alkoxy en C₁ à C₈, (alkoxy en C₁ à C₄)-(alkyle en C₁ à C₄), (alkylthio en C₁ à C₄)-(alkyle en C₁ à C₄) ou halogénalkyle en C₁ à C₆,
R³ représente l'hydrogène, un reste alkyle en C₁ à C₈, alkoxy en C₁ à C₈, (alkoxy en C₁ à C₄) - (alkyle en C₁ à C₄) , (alkylthio en C₁ à C₄)-(alkyle en C₁ à C₄), alcényle en C₂ à C₈, alcynyle en C₂ à C₈, halogénalkyle en C₁ à C₆, halogénalcényle en C₂ à C₆, halogénalcynyle en C₂ à C₆, cycloalkyle en C₃ à C₆, ou un reste phényle
ou phénylalkyle, chacun éventuellement substitué,
**caractérisé en ce qu'**on fait réagir du fluorure d'acide 5-fluoro-1,3-diméthyl-1H-pyrazole-4-carboxylique de formule (II) 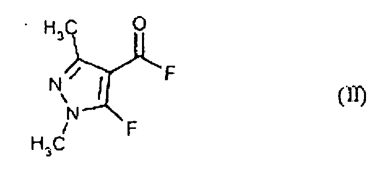 avec des dérivés d'aniline de formule (III) 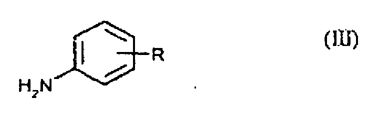 dans laquelle R a les définitions indiquées ci-dessus,
en l'absence d'un accepteur d'acide.

2. Procédé suivant la revendication 1, **caractérisé en ce qu'**on opère à des températures de 120°C à 150°C.

3. Procédé suivant la revendication 1 ou 2, **caractérisé en ce qu'**on utilise des dérivés d'aniline de formule (III-1) 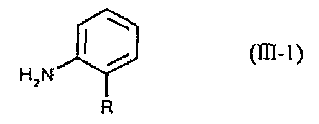 dans laquelle R a les définitions indiquées dans la revendication 1.

4. Procédé suivant la revendication 1, 2 ou 3, **caractérisé en ce qu'**on utilise la 2-(1,3-diméthylbutyl)-phénylamine comme dérivé d'aniline de formule (III).
